(19) **Europäisches Patentamt European Patent Office Office européen des brevets**

(11) **EP 4 527 287 A1**

(12) **EUROPEAN PATENT APPLICATION**
published in accordance with Art. 153(4) EPC

(43) Date of publication:
26.03.2025 Bulletin 2025/13

(21) Application number: 22942854.5

(22) Date of filing: 11.11.2022

(51) International Patent Classification (IPC):
*A61B 5/00* (2006.01)        *G06T 7/00* (2017.01)
*G06T 7/60* (2017.01)

(52) Cooperative Patent Classification (CPC):
A61B 5/00; G06T 7/00; G06T 7/60

(86) International application number:
PCT/KR2022/017758

(87) International publication number:
WO 2023/224191 (23.11.2023 Gazette 2023/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.05.2022  KR 20220062212
04.08.2022  KR 20220097349

(71) Applicant: **Ahn, Ga Ram**
**Seoul 06909 (KR)**

(72) Inventor: **Ahn, Ga Ram**
**Seoul 06909 (KR)**

(74) Representative: **Keilitz Haines & Partner**
**Patentanwälte PartGmbB**
**Triftstraße 5**
**80538 München (DE)**

(54) **SKIN SURFACE DISPLACEMENT-BASED DEVICE FACTOR CALCULATION SYSTEM**

(57)    Provided is a device factor calculation system based on skin surface displacement for calculating a distribution of displacement and a size of an actually contracted dermal area according to derivation of displacement data around a skin dermal coagulation spot from images before and after photographing a dermal coagulation spot formation area of skin, including a preprocessor configured to take captured images before and after a skin procedure through an energy-based medical device as input to preprocess landmarks of skin as location data in the captured images before and after the skin procedure, a displacement calculator configured to derive landmark displacement data from landmark location data of the skin derived by the preprocessor, and to derive location data of a contraction center O based on the landmark displacement data, and a boundary deriver configured to derive a boundary of a contraction zone from the contraction center O in the skin procedure.

FIG. 1

EP 4 527 287 A1

## Description

[Technical Field]

The present invention relates to a device factor calculation system based on skin surface displacement, and more particularly to a device factor calculation system based on skin surface displacement configured to derive displacement data around a coagulation spot from images captured before and after formation of the coagulation spot on skin, thereby calculating a size and shape of a contraction zone caused by the coagulation spot.

[Background Art]

The number of consumers visiting dermatologists due to skin sagging and undergoing skin procedures, such as lifting procedures, has been continuously increasing since such skin procedures have relatively short recovery periods compared to plastic surgeries and effectively enable facial wrinkle lifting (that is, skin area contraction).

Here, an energy-based medical device for skin area contraction forms a coagulation zone on the skin around a target point. In more detail, the reticular dermis includes spiral collagen bundles, which are arranged in a direction parallel to the skin surface, and when each bundle is thermally coagulated, a spiral structure unwinds and becomes tangled to be shorter. When energy is applied to one point of a reticular dermis layer to form a coagulation spot, the reticular dermis layer within a range of coagulation around the point contracts in a direction toward the coagulation spot in a plane parallel to the skin surface to form a contraction zone. When several such coagulation spots are formed, an immediate area reduction of a zone including the coagulation spots is expected, and thus the energy-based medical device uses this principle to induce a cosmetic effect by flattening the skin.

However, there is a limitation that the effect is not constant when the procedure is performed on actual patients. In other words, efficiency of planar contraction of the skin through induction of multiple dermal coagulation spots is not consistent in reality. When coagulation ranges do not contact each other and are separated from each other, non-coagulated skin therebetween increases (compensatory dilatation) and the overall area reduction effect is reduced. Conversely, when overlap between coagulation zones occurs, temperature excessively rises (for example, to about 80 degrees Celsius or more), and thus there is a risk of covalent bonds inside collagen molecules breaking down, causing dissolution thereof. In addition, even when adjacent coagulation ranges are to be in contact, a contraction zone caused by one coagulation spot varies depending on several factors such as a patient receiving the procedure, a region, output of the device, a depth, and an angle, and there is no method to instantly identify a size and shape of a contraction zone induced by one coagulation spot to date.

Therefore, theoretically, in order to optimize skin area reduction effects using an energy-based device, technology capable of finding a size and shape of a contraction zone generated by one coagulation spot is needed. That is, it should be possible to adjust a location where adjacent coagulation spots are generated or output thereof based on information on the size and shape of the contraction zone generated by each coagulation spot found by the above technology. However, at present, in this procedure, the size and shape of the contraction zone generated by each coagulation spot are estimated and a distance between coagulation spots and output adjustment of the device are determined depending on intuition of an operator, and thus there are limitations in terms of efficiency, reproducibility, and safety of the procedure.

[Disclosure]

[Technical Problem]

Therefore, the present invention has been made in view of the above problems, and it is an object of the present invention to provide a device factor calculation system based on skin surface displacement configured to use skin surface information before and after formation of a coagulation spot of skin as input to calculate a size of a contraction zone caused by the coagulation spot.

[Technical Solution]

In accordance with the present invention, the above and other objects can be accomplished by the provision of a device factor calculation system based on skin surface displacement including a preprocessor configured to take captured images before and after a skin procedure through an energy-based medical device as input to preprocess landmarks of skin as location data in the captured images before and after the skin procedure, a displacement calculator configured to derive landmark displacement data from landmark location data of the skin derived by the preprocessor, and to derive location data of a contraction center O based on the landmark displacement data and a boundary deriver configured to derive a boundary of a contraction zone from the contraction center O in the skin procedure.

The energy-based medical device may be configured to apply energy to one focus of the skin to cause a local temperature rise, thereby generating a contraction zone based on the one focus of the skin, and include high-frequency, laser, and high-intensity focused ultrasound (HIFU) devices.

The landmarks of the skin may include dots or lines marked with pigment, and sweat glands, hair glands, sebaceous glands, pigmented lesions, moles, skin tumors, blood vessels, and wrinkle lines on a skin surface as reference points enabling tracking of positional change of a skin procedure target part before and after the procedure in the captured images before and after the skin procedure through the energy-based medical device.

The preprocessor may be configured to calculate and store location data A of landmarks before the skin procedure on a skin surface in the captured image before the skin procedure, and calculate and store location data B of landmarks after the skin procedure on the skin surface in the captured image after the skin procedure according to movement of locations of the landmarks before the skin procedure on the skin surface to the contraction center O by the skin procedure.

The preprocessor may be configured to represent the location data A of the landmarks before the skin procedure on the skin surface as $A[(i, j)ij]$, and represent the location data B of the landmarks after the skin procedure on the skin surface as $B[(i', j')ij]$.

The displacement calculator may calculate, as the landmark displacement data D, a displacement vector reflecting a result of movement from location data before the skin procedure to location data after the procedure based on the landmark location data of the skin derived by the preprocessor, and the landmark displacement data D may be represented as $[(i' - i, j' - j)ij]$, where $i'$ and $j'$ are a row $i'$ and a column $j'$ of $B[(i', j')ij]$, and $i$ and $j$ are a row $i$ and a column $j$ of $A[(i, j)ij]$.

The boundary deriver may include a graph deriver configured to derive a displacement graph of each point moving toward the contraction center O for each location on a straight line passing through, at an angle $\Theta$, the contraction center O corresponding to a location of a focus to which the energy-based medical device applies energy based on the landmark displacement data, and a boundary deriver configured to calculate a distance R from the contraction center O to the boundary of the contraction zone based on graph analysis result data derived by the graph deriver.

The graph deriver may set the location data of the contraction center O corresponding to a location of a focus to which the energy-based medical device applies energy based on the landmark displacement data D, the location data of the contraction center O may be a location of a point to which landmark points on a skin surface are commonly directed during skin coagulation according to the procedure, and a location of an intersection point of straight lines, which are obtained by points corresponding to respective elements $(i, j)ij$ of location data A of landmarks before the skin procedure on the skin surface each directed in a direction of an element $\vec{\delta}$ ij vector of the landmark displacement data D, may be set to $(io, jo)$.

The graph deriver may perform parallel translation by $-io$ on an i-axis and $-jo$ on a j-axis with respect to a contraction center O $(io, jo, 0)$ in the skin procedure, and perform rotation by the angle $\Theta$ at which the contraction center O in the skin procedure is passed through on a k-axis, thereby deriving a graph of k with respect to i in an ik-plane $(j = 0)$.

The boundary deriver may be configured to derive a graph of a regression curve of a distance by which each point moves toward the contraction center O in the skin procedure for each location on a horizontal line $(\Theta = 0)$ passing through the contraction center O in the skin procedure, and calculate a distance from the contraction center O to the boundary R of the contraction zone from the captured image before the procedure based on a fact that the distance is close to a distance from the contraction center O to a point where a slope of a tangent becomes 0 on the regression curve.

[Advantageous effects]

According to the device factor calculation system based on skin surface displacement described above:

First, it is possible to derive displacement of each point on a skin surface that contracts when one focal point of skin is treated using an energy-based medical device.

Second, it is possible to derive a location of a contraction center caused by the procedure based on photographs taken before and after the procedure.

Third, it is possible to quantitatively express an aspect of skin contraction by the procedure based on the derived data.

Fourth, it is possible to quantitatively express an aspect of skin contraction, which varies according to a patient undergoing the procedure and a part thereof and according to a type and output of the device, whenever the system is applied.

Fifth, it is possible to generate data necessary for deriving an optimal next procedure location or procedure output during the procedure based on mathematical calculation rather than intuition of the operator.

Sixth, it is possible to combine distribution information on a contraction aspect of skin in several parts in one procedure target to calculate and propose a twodimensional (2D) arrangement and order of several procedure points for realizing a change to desired appearance through manipulation of a location of each point on the skin.

[Description of Drawings]

FIG. 1 is a configuration diagram of the present invention;
FIGs. 2 and 3 are schematic diagrams of skin landmarks, a helical structure diagram of collagen in skin, and exemplary views of contraction when a coagulation zone is generated in the skin;
FIG. 4 is a schematic diagram of a skin contraction zone generated by a coagulation range of a reticular dermis layer of the skin when energy is irradiated to the skin using the energy-based medical device;

FIGs. 5 and 6 are diagrams describing that safety and area reduction efficiency may vary depending on the distance between adjacent coagulation spots when reducing the area of the skin by generating several coagulation spots;

FIG. 7 is a schematic diagram of a process of calculating location data by a preprocessor 100 according to an embodiment of the present invention;

FIG. 8 is schematic diagram of a process of calculating displacement data D by the displacement calculator 200 according to an embodiment of the present invention;

FIG. 9 is a configuration diagram of a boundary deriver 300 according to an embodiment of the present invention;

FIG. 10 is a process diagram of calculating a location of a contraction center O by a graph deriver 300a according to an embodiment of the present invention;

FIG. 11 is a graph of the graph deriver 300a according to an embodiment of the present invention;

FIG. 12 is a process diagram of calculating a size and shape of the contraction zone by a boundary deriver 300b according to an embodiment of the present invention; and

FIG. 13 is a calculation process diagram when an angle of a straight line passing through a contraction center is 0 ($\Theta = 0$) according to an embodiment of the present invention.

[Best Mode]

A device factor calculation system based on skin surface displacement according to embodiments of the present invention will be described in detail with reference to the accompanying drawings. Since the present invention may undergo various changes and have various forms, specific embodiments will be illustrated in the drawings and described in detail in the text. However, this is not intended to limit the present invention to a specific form disclosed, and should be understood to include all modifications, equivalents, or substitutes included in the spirit and scope of the present invention. Like reference numerals have been used for like elements throughout the description of each figure. In the accompanying drawings, dimensions of structures are illustrated to be enlarger than actual ones for clarity of the present invention, or reduced compared to actual ones for understanding of schematic configurations.

In addition, even though terms such as first and second may be used to describe various components, the components should not be limited by the terms. The terms are only used for the purpose of distinguishing one component from another. For example, a first component may be referred to as a second component, and similarly, the second component may be may be referred to as the first component, without departing from the scope of the present invention. Meanwhile, unless defined otherwise, all terms used herein, including technical or scientific terms, have the same meaning as commonly understood by a person of ordinary skill in the art to which the present invention pertains. Terms such as those defined in commonly used dictionaries should be interpreted as having meanings consistent with meanings in the context of the related art, and should not be interpreted as having ideal or excessively formal meanings unless explicitly defined in the present application.

The present invention relates to a device factor calculation system based on skin surface displacement, and more particularly to a device factor calculation system based on skin surface displacement configured to derive displacement data around a coagulation spot from images captured before and after formation of the coagulation spot on the skin, thereby calculating a size and shape of a coagulation range caused by the coagulation spot. Therefore, the present invention calculates the size and shape of the coagulation range through skin surface information before and after generation of the coagulation spot, thereby aiding in optimization of procedure factors such as a distance between adjacent coagulation spots and output of the device during a cosmetic procedure using an energy-based medical device.

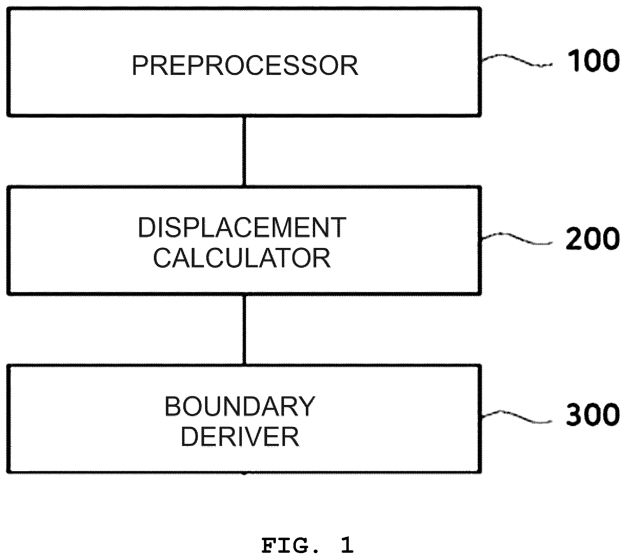

FIG. 1 is a configuration diagram of the present invention.

Referring to FIG. 1, a device factor calculation system based on skin surface displacement includes a preprocessor 100, a displacement calculator 200, and a boundary deriver 300. The preprocessor 100 uses captured images before and after a skin procedure through an energy-based medical device as input to preprocess skin landmarks as location data in the captured images before and after the skin procedure. The displacement calculator 200 derives landmark displacement data from landmark location data of skin derived from the preprocessor 100, and derives location data of a contraction center O based on the landmark displacement data, and the boundary deriver 300 derives a boundary of a contraction zone from the contraction center O in the skin procedure.

That is, in more detail, the preprocessor 100 takes, as input, captured images before and after a procedure, in which the energy-based medical device generates a coagulation spot by applying energy to one focal point of the skin, to calculate (preprocess) skin landmark location data before and after generation of the coagulation spot. Thereafter, the displacement calculator 200 calculates displacement data of the skin landmark based on a change in the landmark location data before and after the procedure, and calculates and derives location data of the contraction center O from the landmark location data and the displacement data. Finally, the boundary deriver 300 calculates a distance R from the contraction center O to a contraction zone boundary based on the location data, the displacement data, and the location data of the contraction center O.

Here, a medical device configured to apply energy to one focus of the skin to cause a local temperature rise, thereby generating a contraction zone based on the one focus of the skin, and transfer physical energy to body tissue to change the tissue is given as an example of the energy-based medical device, and examples thereof include high-frequency, laser, and high-intensity focused ultrasound (HIFU) devices.

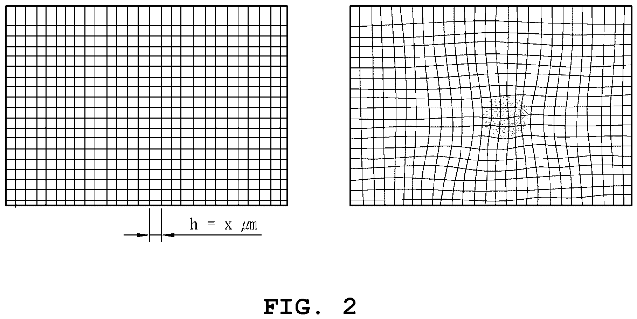

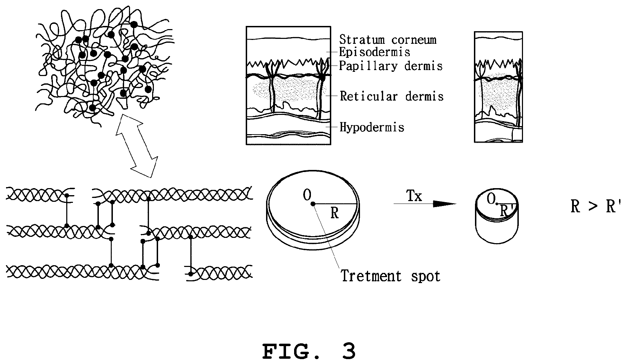

FIGs. 2 and 3 are schematic diagrams of skin landmarks, a helical structure diagram of collagen in skin, and exemplary views of contraction when a coagulation zone is generated in the skin.

Referring to FIG. 2, the skin landmarks are reference points enabling tracking of positional change of a skin procedure target part before and after the procedure in captured images before and after the skin procedure through the energy-based medical device, and include dots or lines marked with pigment, and sweat glands, hair glands, sebaceous glands, pigmented lesions, moles, skin tumors, blood vessels, and wrinkle lines on a skin surface. Referring to FIG. 3, there is a reticular dermis layer filled with collagen bundles in a deep layer of the skin surface. The reticular dermis layer includes spiral collagen bundles, and these collagen bundles are arranged in a direction parallel to the skin surface. When each of the bundles is thermally solidified, the bundle becomes shorter as a spiral structure unwinds and tangles. When a coagulation spot is formed by applying energy to one point of the reticular dermis layer, the reticular dermis layer within a range of coagulation around the point shrinks in a direction toward the corresponding coagulation spot in a plane parallel to the skin surface. Further, a contraction zone caused by one coagulation spot varies depending on several factors such as a patient undergoing the procedure, a part thereof, output of the device, a depth, and an angle.

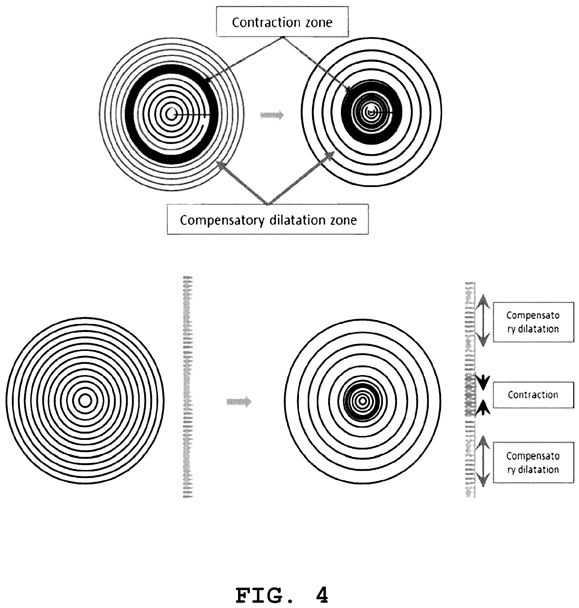

FIG. 4 is a schematic diagram of a skin contraction zone generated by a coagulation range of the reticular dermis layer of the skin when energy is irradiated to the skin using the energy-based medical device.

Referring to FIG. 4, when energy is applied to one focus of the reticular dermis by the energy-based medical device to form a coagulation spot, a contraction zone is formed within a distance R that may vary depending on the angle Θ based on the contraction center O. Within the contraction zone, each point on the skin surface moves toward the contraction center O, and as the density of the points increases, a distance between adjacent points becomes shorter. In addition, as the contraction zone is generated, the distance R from the contraction center O to the contraction zone boundary becomes shorter (R' < R). Conversely, at each point outside the distance R, that is, at a point outside the contraction zone, as the tension increases in a direction toward the contraction center O due to contraction generated within the contraction zone, a compensatory dilatation zone in which the density of each point on the skin surface is lowered is generated.

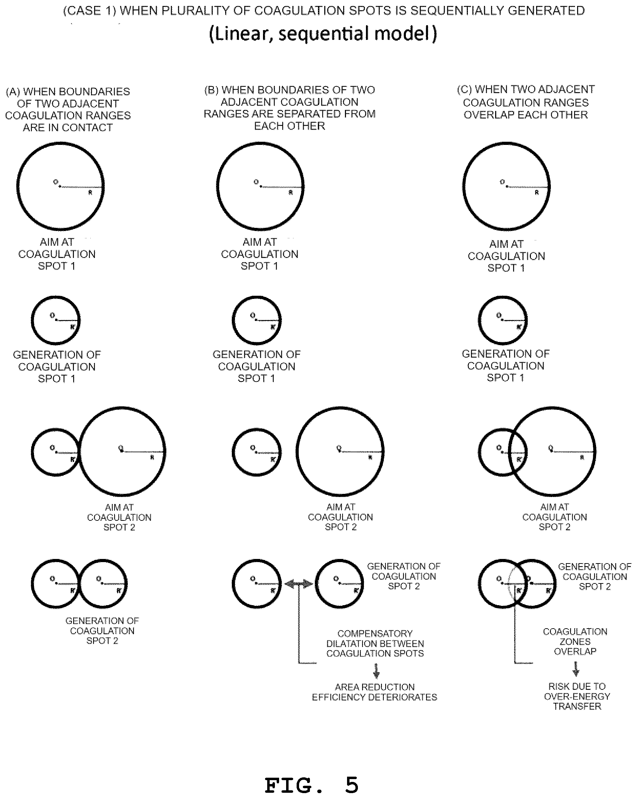

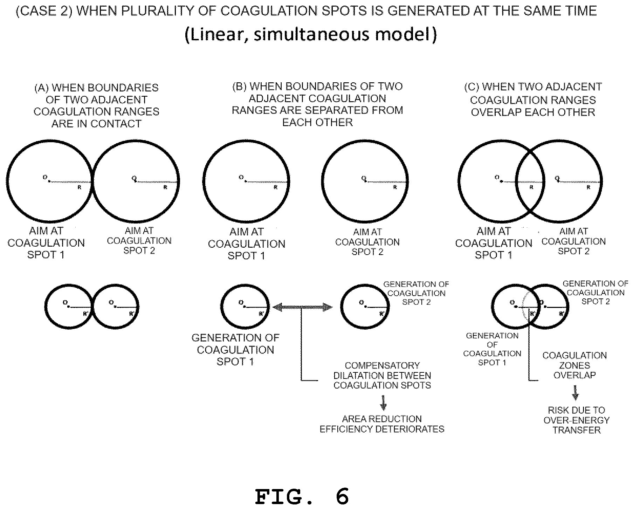

FIGs. 5 and 6 describe that safety and area reduction efficiency may vary depending on the distance between adjacent coagulation spots when reducing the area of the skin by generating several coagulation spots.

Referring to FIGs. 5 and 6, when coagulation ranges are not in contact with each other and are separated from each other, non-coagulated skin therebetween increases (compensatory dilatation), and thus the overall area reduction effect is reduced. Conversely, when overlapping between coagulation zones occurs, there is a risk that temperature may excessively rise (for example, about 80 degrees Celsius or more) and covalent bonds inside collagen molecules may be broken down, causing dissolution thereof.

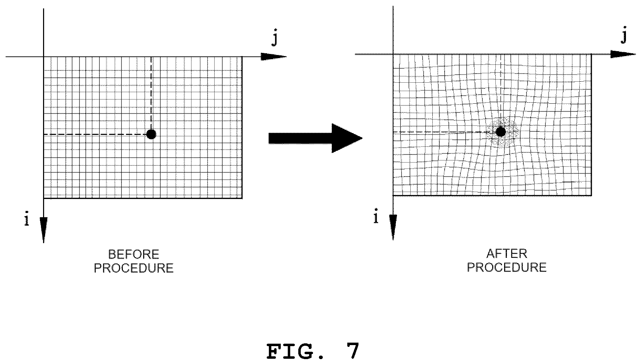

FIG. 7 is a process of calculating location data by the preprocessor 100 according to an embodiment of the present invention.

Referring to FIG. 7, the preprocessor 100 calculates and stores location data A of landmarks on the skin surface before the skin procedure from the image captured before the skin procedure, and calculates and stores location data B of landmarks on the skin surface after the skin procedure from the image captured after the skin procedure according to movement of locations of the landmarks on the skin surface before the skin procedure to the contraction center O due to the skin procedure.

Thereafter, the location data A of the landmarks on the skin surface before the skin procedure is represented as A[(i, j)ij], and the location data B of the landmarks on the skin surface after the skin procedure is represented as B[(i', j')ij]. That is, in more detail, as an embodiment of the present invention, in order to calculate each of the location data A of the landmarks on the skin surface before the skin procedure and the location data B of the landmarks on the skin surface after the skin procedure, pixel coordinates in the captured images before and after the skin procedure are expressed in a 2D Cartesian coordinate system having the origin at a top left vertex of the images, an i-axis directed downward along a vertical line, and a j-axis directed rightward along a horizontal line, and represented by the following matrix A in which coordinates (i, j) of each pixel on the image data correspond to an element in a row i and a column j.

$$A = \begin{bmatrix} (1,1) & (1,2) & (1,3) & \\ (2,1) & (2,2) & (2,3) & \cdots \\ (3,1) & (3,2) & (3,3) & \\ & & \vdots & \ddots \end{bmatrix}$$

The location data B of the landmarks on the skin surface after the skin procedure may be represented by a matrix [(i', j')ij] having, as an element, coordinates (i', j') of a point at which each element [(i, j)ij] of the matrix A arrives after contraction.

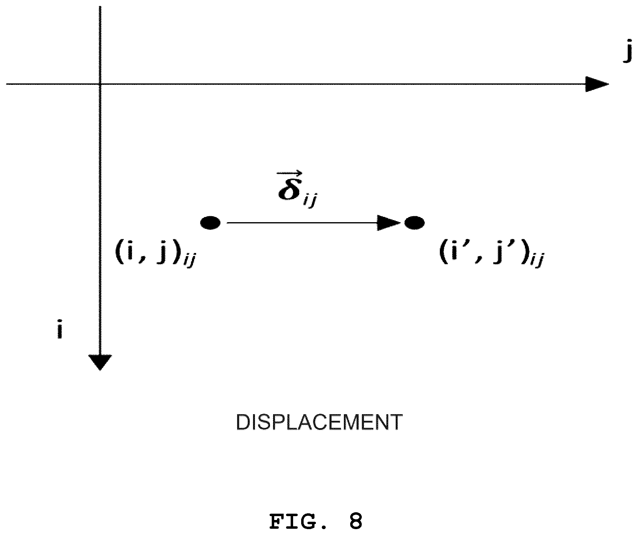

FIG. 8 is a state in which the displacement calculator 200 according to an embodiment of the present invention calculates displacement data D.

Referring to FIG. 8, the displacement calculator 200 represents a result obtained by movement of the location data of the skin landmarks derived by the preprocessor from the location data A before the skin procedure to the location data after the procedure by displacement data D expressed as a vector $\vec{\delta}ij$. Here, as an example of the displacement data D, D = [(i' - i, j' - j)ij] = [$\delta$ij].

(Here, i' and j' are a row i' and a column j' of B[(i', j')ij], and i and j are a row i and a column j of A[(i, j)ij].

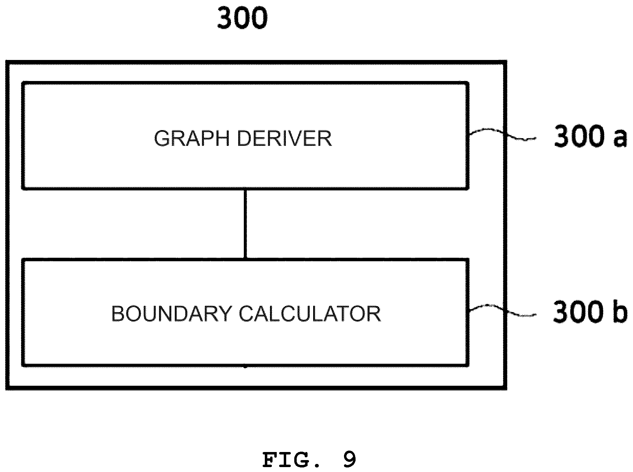

FIG. 9 is a configuration diagram of the boundary deriver 300 according to an embodiment of the present invention.

Referring to FIG. 9, the boundary deriver 300 includes a graph deriver 300a and a boundary deriver 300b. The graph deriver 300a derives a displacement graph of each of landmarks on straight lines passing through the contraction center O after the procedure with respect to a distance from the contraction center O before the procedure based on the landmark location data A before the procedure, location data of the contraction center O, and the displacement data D. The boundary deriver 300b calculates the distance R from the contraction center O to the contraction zone boundary based on graph analysis result data derived by the graph deriver 300a.

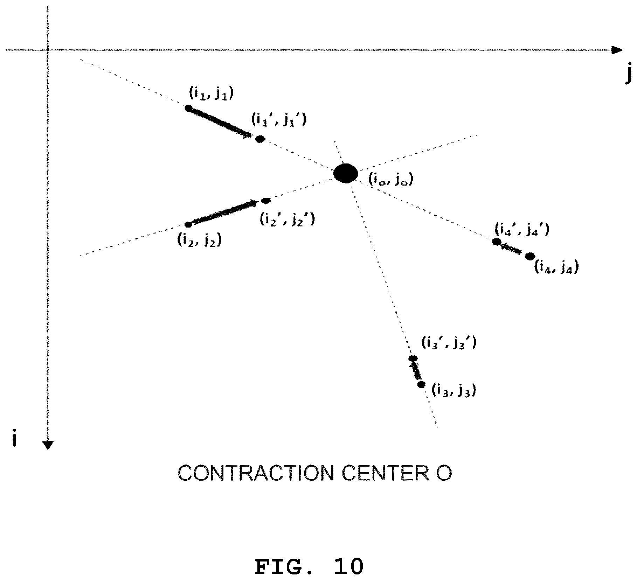

FIG. 10 is process of calculating a location of the contraction center O by the graph deriver 300a according to an embodiment of the present invention.

Referring to FIG. 10, the graph deriver 300a may calculate location data of the contraction center O based on the location data A before the procedure and the displacement data D even when the location of the contraction center O is not known. That is, in more detail, the graph deriver 300a sets the location data of the contraction center O corresponding to a location of a focus to which the energy-based medical device applies energy based on the landmark displacement data D, the location data of the contraction center O is a location of a point to which landmark points on the skin surface are commonly directed during skin coagulation according to the procedure, and a location of an intersection point of straight lines, which are obtained by points corresponding to the respective elements (i, j)ij of the matrix A each directed in a direction of an element $\vec{\delta}ij$ vector of the landmark displacement data D, is set to (io, jo).

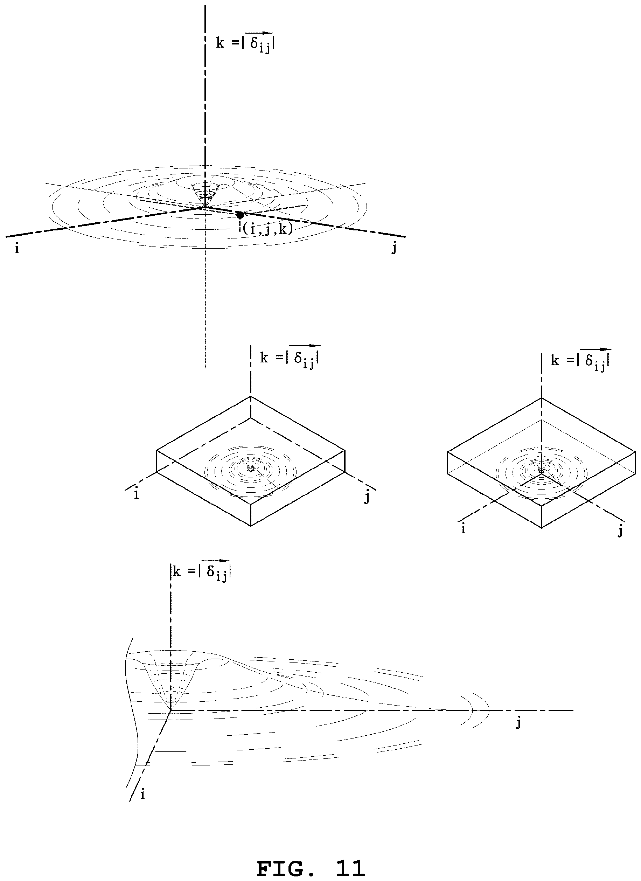

FIG. 11 is a graph of the graph deriver 300a according to an embodiment of the present invention.

Referring to FIG. 11, the graph deriver 300a may derive a graph of a distance (displacement) of each point moving toward the contraction center O for each point on a straight line passing through the contraction center O at a desired angle Θ in the captured image before the procedure using the location data and the displacement data as input. In embodiments of the present invention, the graph deriver 300a derives a displacement graph after the procedure with respect to a distance of each of landmarks on a straight line, which passes through the contraction center O in the skin procedure at the desired angle Θ, from the contraction center O before the procedure, performs parallel translation by -io on the i-axis and -jo on the j-axis with respect to the contraction center O (io, jo, 0) in the skin procedure, and performs rotation by the angle Θ at which the contraction center O in the skin procedure is passed through on a k-axis, thereby deriving a graph of k with respect to i in an ik-plane (j = 0). That is, in more detail, in a three-dimensional (3D) Cartesian coordinate system obtained by adding the k-axis to the Cartesian coordinate system of the i-axis and the j-axis, a set of points [i, j, |$\vec{\delta}_{ij}$|], where values corresponding to the i-axis and the j-axis are set to values i and j of an element [(i, j)ij] of the matrix A, and a value corresponding to the k-axis is set to the magnitude |$\vec{\delta}_{ij}$| of a vector element of the data D corresponding to the element [(i, j)] of matrix A, may be represented by a graph, which may be moved by -io on the i-axis and -jo on the j-axis to place a center of symmetry thereof on the k-axis, then rotated by the angle Θ around the k-axis, and then expressed as a graph of k with respect to i in the ik-plane (j = 0). In the process of deriving a graph, a location of a coagulation center O is not given, and a description has been given of a method capable of calculating a displacement distribution graph for all straight lines passing through the unknown coagulation center O. However, when the location of the coagulation center O is previously known, or only a displacement graph on one straight line is to be obtained, the corresponding step in the process of deriving a graph may be omitted. Then, the boundary deriver 300b calculates the size and shape of the contraction zone.

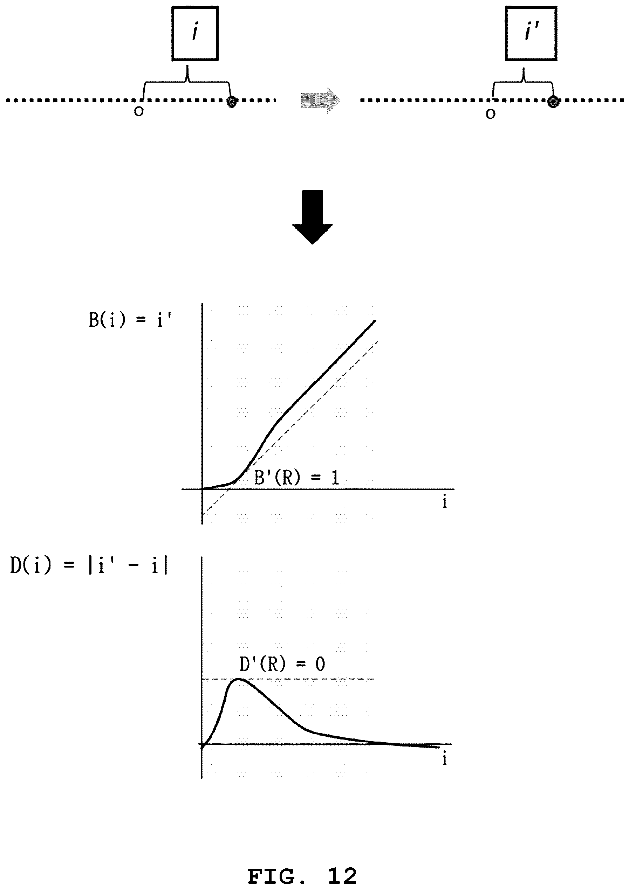

FIG. 12 is a process of calculating a size and shape of the coagulation center contraction zone by the boundary deriver 300b according to an embodiment of the present invention.

Referring to FIG. 12, the boundary deriver 300b derives a graph of a regression curve of a distance by which each point moves toward the contraction center O in the skin procedure for each location on the horizontal line (Θ = 0) passing through the contraction center O in the skin procedure, and calculates a distance from the contraction center O to the boundary R of the contraction zone from the captured image before the procedure based on the fact that the distance is close to a distance from the contraction center O to a point where a slope of a tangent becomes 0 on the regression curve. Therefore, it is possible to derive a regression curve for a scatter plot including discrete values obtained from actual observations.

As an embodiment of the present invention, the graph of the distance (displacement) by which each point moves toward the contraction center O for each point on the straight line passing through the contraction center O at the desired angle Θ in the captured image before the procedure obtained by the graph deriver 300a regresses to a distribution curve D(i) having positive numbers as elements (Lognormal distribution, Chi-square distribution, F-distribution, and other distribution curves having positive numbers as elements). Thus, with respect to a distance (i) from the contraction center O to an element of the location data A before the procedure derived by the preprocessor 100, a graph of a distance (i') to an element of the location data B after the procedure corresponding to the element may be represented by B(i) = i - D(i). Therefore, the boundary deriver 300b may derive a size and shape of the contraction zone based on the fact that an i-coordinate at a point where a slope B'(i) of a tangent is 1 is close to a distance from the contraction center O to the boundary R of the contraction zone in the same direction on the curve B(i). Here, the graph of the distance (i') to the element of the location data B after the procedure corresponding to the element of the location data A before the procedure with respect to the distance (i) from the contraction center O to the element of the location data A before the procedure derived by the preprocessor 100 regresses to the curve B(i).

Further, in an embodiment of the present invention, when a cross section of the displacement graph derived by the graph deriver 300a regresses to a distribution curve having positive numbers as elements, a mode of the distribution, that is, an i-coordinate at a highest point where a slope of a tangent is 0 is close to a distance from the contraction center O to the boundary R of the contraction zone on a straight line passing through the contraction center O at an angle corresponding to the cross section, and thus the boundary deriver 300b may calculate the size and shape of the contraction zone. Since the distance from the contraction center O to the boundary R of the contraction zone may vary depending on the direction of the straight line passing through the contraction center O, the 3D graph derived by the graph deriver 300a may be rotated by a desired angle around the contraction center O to cause the boundary deriver 300b to calculate a distance to the boundary R of the contraction zone in all directions with respect to the contraction center O, which corresponds to obtaining the size and shape of the contraction zone.

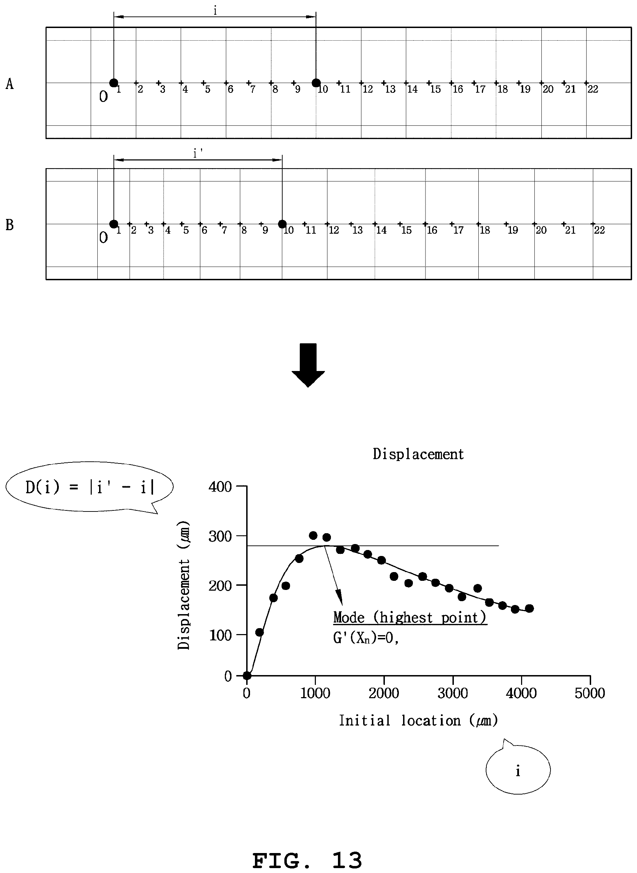

FIG. 13 is a calculation process when an angle of a straight line passing through a contraction center is 0 ($\Theta = 0$) according to an embodiment of the present invention.

Referring to FIG. 13, the location data A before the procedure and the location data B after the procedure may be obtained based on landmark location data preprocessed by the preprocessor 100. Thereafter, the displacement calculator 200 calculates displacement D(i), which is a change in location data for each coagulation point of the skin, based on the location data acquired by the preprocessor 100. The location of the contraction center O is previously known or is obtained through calculation by the displacement calculator 200 as described in the embodiment of the graph deriver 300a, and then a graph is derived by the graph deriver 300a through this value. A cross section of a graph moved to the origin of a virtual coordinate system to calculate the size and shape of the contraction zone by the boundary deriver 300b regresses to a lognormal distribution with a regression coefficient of 0.6 or more ($R^2 = 0.9562$). Therefore, it may be determined that the scatter plot is not different from a population following the lognormal distribution of

$$D(i) = \frac{1}{i\sigma\sqrt{2\pi}} e^{\left\{ -\frac{(\ln i - \mu)^2}{2\sigma^2} \right\}}.$$

In the regressing displacement distribution curve D(i), a highest point corresponds to a mode, and a slope D'(i) of a tangent at the point is 0. In the lognormal distribution, a mode is (e being a natural constant, $\mu$ being a mean, and $\sigma$ being a standard deviation), and thus a value i and a value i' satisfying D'(i) = 0 may be obtained by a probability density function equation. Therefore, by providing information on the shape and size of the contraction zone derived through the boundary deriver 300 to the operator or device, it is possible to implement an optimal state illustrated in the drawings of FIGs. 5 and 6, that is, a state in which boundaries of the contraction zones are in contact.

According to the device factor calculation system based on skin surface displacement described above, there are the following effects. First, it is possible to derive displacement of each point on a skin surface that contracts when one focal point on the skin is treated using an energy-based medical device. Second, it is possible to derive a location of a contraction center caused by the procedure based on photographs taken before and after the procedure. Third, it is possible to quantitatively express an aspect of skin contraction by the procedure based on the derived data. Fourth, it is possible to quantitatively express an aspect of skin contraction, which varies according to a patient undergoing the procedure and a part thereof and according to a type and output of the device, whenever the system is applied. Fifth, it is possible to generate data necessary for deriving an optimal next procedure location or procedure output during the procedure based on mathematical calculation rather than intuition of the operator. Sixth, it is possible to combine distribution information on a contraction aspect of skin in several parts in one procedure target to calculate and propose a 2D arrangement and order of several procedure points for realizing a change to desired appearance through manipulation of a location of each point on the skin.

Even though the detailed description of the present invention described above has been given with reference to preferred embodiments of the present invention, those skilled in the art or those having ordinary knowledge in the art may understand that the present invention may be variously modified and changed within the scope not departing from the spirit and technical scope of the present invention described in the claims to be described later.

[Industrial Applicability]

The device factor calculation system based on skin surface displacement may be installed in various energy-based medical devices such as fractional fine needle radiofrequency, fractional laser, and high-intensity focused ultrasound, and cosmetic effects may be maximized through automatic calculation of contraction of a skin plane and a calculated value.

**Claims**

1. A device factor calculation system based on skin surface displacement comprising:

   a preprocessor configured to take captured images before and after a skin procedure through an energy-based medical device as input to preprocess landmarks of skin as location data in the captured images before and after the skin procedure;
   a displacement calculator configured to derive landmark displacement data from landmark location data of the skin derived by the preprocessor, and to derive location data of a contraction center O based on the landmark displacement data; and
   a boundary deriver configured to derive a boundary of a contraction zone from the contraction center O in the skin procedure.

2. The device factor calculation system based on skin surface displacement according to claim 1, wherein the energy-based medical device is configured to:

   apply energy to one focus of the skin to cause a local temperature rise, thereby generating a contraction zone based on the one focus of the skin, and
   include high-frequency, laser, and high-intensity focused ultrasound (HIFU) devices.

3. The device factor calculation system based on skin surface displacement according to claim 1, wherein the landmarks of the skin comprise dots or lines marked with pigment, and sweat glands, hair glands, sebaceous glands, pigmented lesions, moles, skin tumors, blood vessels, and wrinkle lines on a skin surface as reference points enabling tracking of positional change of a skin procedure target part before and after the procedure in the captured images before and after the skin procedure through the energy-based medical device.

4. The device factor calculation system based on skin surface displacement according to claim 1, wherein the preprocessor is configured to:

   calculate and store location data A of landmarks before the skin procedure on a skin surface in the captured image before the skin procedure; and
   calculate and store location data B of landmarks after the skin procedure on the skin surface in the captured image after the skin procedure according to movement of locations of the landmarks before the skin procedure on the skin surface to the contraction center O by the skin procedure.

5. The device factor calculation system based on skin surface displacement according to claim 4, wherein the preprocessor is configured to:

   represent the location data A of the landmarks before the skin procedure on the skin surface as $A[(i, j)ij]$, and
   represent the location data B of the landmarks after the skin procedure on the skin surface as $B[(i', j')ij]$.

6. The device factor calculation system based on skin surface displacement according to claim 5, wherein:

   the displacement calculator calculates, as the landmark displacement data D, a displacement vector reflecting a result of movement from location data before the skin procedure to location data after the procedure based on the landmark location data of the skin derived by the preprocessor, and
   the landmark displacement data D is represented as $[(i' - i, j' - j)ij]$, where $i'$ and $j'$ are a row $i'$ and a column $j'$ of $B[(i', j')ij]$, and $i$ and $j$ are a row $i$ and a column $j$ of $A[(i, j)ij]$.

7. The device factor calculation system based on skin surface displacement according to claim 1, wherein the boundary deriver comprises:

   a graph deriver configured to derive a displacement graph of each point moving toward the contraction center O for each location on a straight line passing through, at an angle $\Theta$, the contraction center O corresponding to a location of a focus to which the energy-based medical device applies energy based on the landmark displacement data; and
   a boundary deriver configured to calculate a distance R from the contraction center O to the boundary of the contraction zone based on graph analysis result data derived by the graph deriver.

8. The device factor calculation system based on skin surface displacement according to claim 7, wherein:

the graph deriver sets the location data of the contraction center O corresponding to a location of a focus to which the energy-based medical device applies energy based on the landmark displacement data D,

the location data of the contraction center O is a location of a point to which landmark points on a skin surface are commonly directed during skin coagulation according to the procedure, and

a location of an intersection point of straight lines, which are obtained by points corresponding to respective elements (i, j)ij of location data A of landmarks before the skin procedure on the skin surface each directed in a direction of an element $\vec{\delta}$ij vector of the landmark displacement data D, is set to (io, jo).

9. The device factor calculation system based on skin surface displacement according to claim 7, wherein the graph deriver performs parallel translation by -io on an i-axis and -jo on a j-axis with respect to a contraction center O (io, jo, 0) in the skin procedure, and performs rotation by the angle Θ at which the contraction center O in the skin procedure is passed through on a k-axis, thereby deriving a graph of k with respect to i in an ik-plane (j = 0).

10. The device factor calculation system based on skin surface displacement according to claim 7, wherein the boundary deriver is configured to:

derive a graph of a regression curve of a distance by which each point moves toward the contraction center O in the skin procedure for each location on a horizontal line (Θ = 0) passing through the contraction center O in the skin procedure, and

calculate a distance from the contraction center O to the boundary R of the contraction zone from the captured image before the procedure based on a fact that the distance is close to a distance from the contraction center O to a point where a slope of a tangent becomes 0 on the regression curve.

PREPROCESSOR — **100**

DISPLACEMENT CALCULATOR — **200**

BOUNDARY DERIVER — **300**

FIG. 1

h = x $\mu$m

FIG. 2

Stratum corneum
Episodermis
Papillary dermis

Reticular dermis

Hypodermis

Tretment spot

Tx

R > R'

FIG. 3

Contraction zone

Compensatory dilatation zone

Compensato ry dilatation

Contraction

Compensato ry dilatation

FIG. 4

(CASE 1) WHEN PLURALITY OF COAGULATION SPOTS IS SEQUENTIALLY GENERATED

(Linear, sequential model)

FIG. 5

(CASE 2) WHEN PLURALITY OF COAGULATION SPOTS IS GENERATED AT THE SAME TIME

## (Linear, simultaneous model)

(A) WHEN BOUNDARIES
OF TWO ADJACENT
COAGULATION RANGES
ARE IN CONTACT

(B) WHEN BOUNDARIES OF TWO
ADJACENT COAGULATION
RANGES ARE SEPARATED FROM
EACH OTHER

(C) WHEN TWO ADJACENT
COAGULATION RANGES
OVERLAP EACH OTHER

AIM AT
COAGULATION
SPOT 1

AIM AT
COAGULATION
SPOT 2

AIM AT
COAGULATION
SPOT 1

AIM AT
COAGULATION
SPOT 2

AIM AT
COAGULATION
SPOT 1

AIM AT
COAGULATION
SPOT 2

GENERATION OF
COAGULATION
SPOT 1

GENERATION OF
COAGULATION
SPOT 2

COMPENSATORY
DILATATION BETWEEN
COAGULATION SPOTS

AREA REDUCTION
EFFICIENCY DETERIORATES

GENERATION OF
COAGULATION
SPOT 2

GENERATION
OF
COAGULATION
SPOT 1

COAGULATION
ZONES
OVERLAP

RISK DUE TO
OVER-ENERGY
TRANSFER

# FIG.  6

BEFORE
PROCEDURE

AFTER
PROCEDURE

FIG. 7

DISPLACEMENT

FIG. 8

**300**

300 a

300 b

FIG. 9

CONTRACTION CENTER O

FIG. 10

FIG. 11

$B(i) = i'$

$B'(R) = 1$

$D(i) = |i' - i|$

$D'(R) = 0$

FIG. 12

$$D(i) = |i' - i|$$

Displacement

Mode (highest point)
$G'(X_n)=0,$

i

FIG. 13

## INTERNATIONAL SEARCH REPORT

| International application No. |
| --- |
| **PCT/KR2022/017758** |

### A. CLASSIFICATION OF SUBJECT MATTER

**A61B 5/00**(2006.01)i; **G06T 7/00**(2006.01)i; **G06T 7/60**(2006.01)i

According to International Patent Classification (IPC) or to both national classification and IPC

### B. FIELDS SEARCHED

Minimum documentation searched (classification system followed by classification symbols)

A61B 5/00(2006.01); A61B 18/00(2006.01); A61B 6/00(2006.01); A61B 8/00(2006.01); A61B 8/08(2006.01); A61B 90/00(2016.01); A61N 7/02(2006.01); G06T 7/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 초음파(ultrasound), 이미지(image), 피부(skin), 랜드마크(landmark), 위치(position), 변위(displacement), 경계(boundary)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2018-0115377 A (KOREA UNIVERSITY RESEARCH AND BUSINESS FOUNDATION et al.) 23 October 2018 (2018-10-23) See claim 5. | 1,4,5 |
| Y | | 2,3,6 |
| A | | 7-10 |
| Y | KR 10-2013-0143434 A (SAMSUNG ELECTRONICS CO., LTD.) 31 December 2013 (2013-12-31) See paragraphs [0041] and [0053]; and claim 10. | 2,3,6 |
| A | KR 10-2018-0136475 A (NEUWAVE MEDICAL, INC.) 24 December 2018 (2018-12-24) See entire document. | 1-10 |
| A | KR 10-2188612 B1 (KOLMAR KOREA CO., LTD. et al.) 09 December 2020 (2020-12-09) See entire document. | 1-10 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **23 February 2023** | **23 February 2023** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/017758**

**C. DOCUMENTS CONSIDERED TO BE RELEVANT**

| Category* | Citation of document, with indication, where appropriate, of the relevant passages | Relevant to claim No. |
|---|---|---|
| A | US 10390796 B2 (FAN, Liexiang et al.) 27 August 2019 (2019-08-27)<br>See entire document. | 1-10 |

Form PCT/ISA/210 (second sheet) (July 2022)

**INTERNATIONAL SEARCH REPORT**
Information on patent family members

International application No.

**PCT/KR2022/017758**

| Patent document cited in search report | | | Publication date (day/month/year) | Patent family member(s) | | | Publication date (day/month/year) |
|---|---|---|---|---|---|---|---|
| KR | 10-2018-0115377 | A | 23 October 2018 | KR | 10-1910822 | B1 | 24 October 2018 |
| KR | 10-2013-0143434 | A | 31 December 2013 | US | 2013-0346050 | A1 | 26 December 2013 |
| KR | 10-2018-0136475 | A | 24 December 2018 | BR | 112018071018 | A2 | 12 February 2019 |
| | | | | CN | 109069203 | A | 21 December 2018 |
| | | | | CN | 109069203 | B | 22 June 2021 |
| | | | | EP | 3442456 | A2 | 20 February 2019 |
| | | | | EP | 3442456 | B1 | 09 December 2020 |
| | | | | EP | 3808302 | A1 | 21 April 2021 |
| | | | | ES | 2854935 | T3 | 23 September 2021 |
| | | | | JP | 2019-513481 | A | 30 May 2019 |
| | | | | JP | 2022-000198 | A | 04 January 2022 |
| | | | | JP | 6949873 | B2 | 13 October 2021 |
| | | | | MX | 2018012563 | A | 08 July 2019 |
| | | | | US | 10531917 | B2 | 14 January 2020 |
| | | | | US | 11395699 | B2 | 26 July 2022 |
| | | | | US | 2017-0296268 | A1 | 19 October 2017 |
| | | | | US | 2020-0146750 | A1 | 14 May 2020 |
| | | | | WO | 2017-180877 | A2 | 19 October 2017 |
| | | | | WO | 2017-180877 | A3 | 23 November 2017 |
| KR | 10-2188612 | B1 | 09 December 2020 | None | | | |
| US | 10390796 | B2 | 27 August 2019 | CN | 104688266 | A | 10 June 2015 |
| | | | | CN | 104688266 | B | 07 January 2020 |
| | | | | EP | 2889004 | A1 | 01 July 2015 |
| | | | | EP | 2889004 | B1 | 17 August 2022 |
| | | | | KR | 10-2015-0065158 | A | 12 June 2015 |
| | | | | KR | 10-2101186 | B1 | 16 April 2020 |
| | | | | US | 2015-0150535 | A1 | 04 June 2015 |

Form PCT/ISA/210 (patent family annex) (July 2022)